(19) Europäisches Patentamt
European Patent Office
Office européen des brevets

(11) EP 0 199 451 B1

(12) **EUROPEAN PATENT SPECIFICATION**

(45) Date of publication and mention of the grant of the patent:
**06.03.1996 Bulletin 1996/10**

(51) Int Cl.[6]: **C07H 19/073**, A61K 31/70

(21) Application number: **86301896.6**

(22) Date of filing: **14.03.1986**

(54) **Therapeutic nucleosides**

Therapeutische Nukleoside

Nucléosides thérapeutiques

(84) Designated Contracting States:
**AT BE CH DE FR GB IT LI LU NL SE**

(30) Priority: **16.03.1985 GB 8506868**
**16.03.1985 GB 8506869**
**09.05.1985 GB 8511774**
**09.05.1985 GB 8511775**
**27.09.1985 GB 8523881**

(43) Date of publication of application:
**29.10.1986 Bulletin 1986/44**

(73) Proprietor: **THE WELLCOME FOUNDATION LIMITED**
**London NW1 2BP (GB)**

(72) Inventors:
- **Rideout, Janet Litster**
  **Raleigh North Carolina (US)**
- **Barry, David Walter**
  **Chapel Hill North Carolina (US)**
- **Lehrman, Sandra Nusinoff**
  **Durham North Carolina (US)**
- **St. Clair, Martha Heider**
  **Durham North Carolina (US)**
- **Furman, Phillip Allen**
  **Durham North Carolina (US)**
- **Beacham III, Lowrie Miller**
  **Durham North Carolina (US)**
- **LeBlanc, Harry Sidney**
  **Raleigh North Carolina (US)**
- **Freeman, George Andrew**
  **Cary North Carolina (US)**

(74) Representative: **Garrett, Michael et al**
**The Wellcome Foundation Limited**
**Group Patents and Agreements**
**Langley Court**
**Beckenham Kent BR3 3BS (GB)**

(56) References cited:
**FR-A- 2 134 292**

- **Experimental Cell Research, vol.116, 1978, pages 21-29, Academic Press Inc., SE; C.J.Krieg et al: "Increase in intracisternal A-type particles in friend cells during inhibition of friend virus (SFFV) release by interferon or azidothymidine"**
- **Proc.Nat.Acad.Sci. USA, vol.71, no.12, december 1974, pages 4980-1985; W.Ostertag et al: "Induction of endogenous virus and of thymidine kinase by bromodeoxyuridine in cell cultures transformed by friend virus"**
- **Experimental Cell Research, vol.116, no.1, 1978, pages 31-37, Academic Press Inc., SE; W.Ostertag et al: "Induction of intracisternal A-type particles during friend cell differentiation"**
- **Biochem.Pharm., vol.29, 1980, pages 1849-1852; E.de Clercq et al: "Antiviral, antimetabolic and antineoplastic activities of 2'- or 3'-amino or -azido-substituted deoxyribo-nucleosides"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 199 451 B1

## Description

The present invention relates to a certain 3'-azido-nudeoside, pharmaceutically acceptable derivatives thereof, and their use in therapy, particularly for the treatment or prophylaxis of certain viral and bacterial infections.

It has been discovered that 3'-azido-3'-deoxythymidine possesses a surprisingly high activity against a broad range of viral and bacterial infections (described below) which renders the compound useful in medical and veterinary therapy. Thus, in a first aspect of the present invention, there is provided a compound of formula (I):

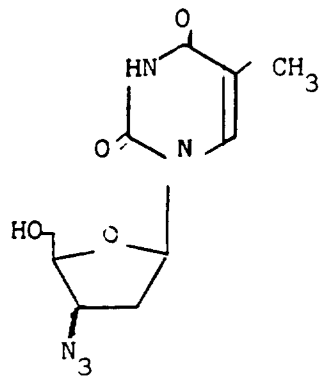

or a pharmaceutically acceptable derivative thereof, for use in therapy, other than for use in the treatment or prophylaxis of a human retroviral infection. The compound of formula (I) and the prharmaceutically acceptable derivatives thereof are hereafter referred to as the compounds according to the invention.

The compounds according to the invention have been found to be particularly useful in the treatment or prophylaxis of gram-negative bacterial infections. Accordingly, the invention also provides the compounds according to the invention for use in the treatment or prophylaxis of gram-negative bacterial infections.

The compounds according to the invention have been found to have good activity against the following bacteria in particular:
Escherichia coli, Salmonella dublin, Salmonella typhosa, Salmonella typhimurium, Shigella flexneii, Citrobacter freundii, Klebsiella pneumoniae, Vibrio cholerae, Vibrio anquillarum, Enterobacter aerogenes, Pasteurella multocida, Haemophilus influenzae, Yersinia enterocolitica, Pasteurella haemolytica, Proteus mirabilis and Proteus vulgaris, the causative organisms of such ailments as travellers' diarrhoea, urinary tract infections, shigellosis, typhoid fever and cholera in humans, as well as animal diseases such as calf neonatal enteritis, pig post-weaning enteritis and chicken colisepticaemia.

Thus, there is further provided the compounds according to the invention for use in the treatment or prophylaxis of infections of any of the above-mentioned bacteria.

Activity of the compounds according to the invention has also been discovered for a broad range of viral infections. There is further provided, therefore, the compounds according to the invention for use in the treatment or prophylaxis of viral infections, other than human retroviral infections.

In vitro testing has shown that the compounds according to the invention have particularly good activity against the following viruses: feline leukaemia virus, equine infectious anaemia virus and other lentiviruses, as well as other human viruses such as hepatitis B virus and Epstein-Barr virus (EBV). The invention accordingly provides the compounds according to the invention for use in the treatment or prophylaxis of any of the above infections.

It will be appreciated that the componds according to the invention may also be used in the manufacture of a medicament for the treatment or prophylaxis of any of the above-mentioned medical or veterinary indications.

By "a pharmaceutically acceptable derivative" is meant any pharmaceutically acceptable salt, ester, or salt of such ester, or any other compound which, upon administration to the recipient, is capable of providing (directly or indirectly) 3'-azido-2',3'-dideoxythymidine or an anti-retrovirally active metabolite or residue thereof. An example of a non-ester compound is the derivative wherein the 5'-C- and 2-C-atoms are linked by an oxygen atom to form an anhydro group.

Preferred esters of the compound of formula (I) include carboxylic acid esters in which the non-carbonyl moiety of the ester grouping is selected from straight or branched chain alkyl, alkoxyalkyl (e.g. methoxymethyl), aralkyl (e.g. benzyl), aryloxyalkyl (e.g. phenoxymethyl), aryl (e.g. phenyl optionally substituted by halogen, $C_{1-4}$ alkyl or $C_{1-4}$ alkoxy); sulphonate esters such as alkyl- or aralkylsulphonyl (e.g. methanesulphonyl); and mono-, di- or tri-phosphate esters.

Any reference to any of the above compounds also includes a reference to a pharmaceutically acceptable salt thereof.

With regard to the above-described esters, unless otherwise specified, any alkyl moiety present advantageously contains 1 to 18 carbon atoms, particularly 1 to 4 carbon atoms. Any aryl moiety present in such esters advantageously comprises a phenyl group.

Examples of pharmaceutically acceptable salts of the compounds of formula (I) and pharmaceutically acceptable derivatives thereof include base salts, eg derived from an appropriate base, such as alkali metal (e.g. sodium), alkaline earth metal (e.g. magnesium) salts, ammonium and $NX_4^+$ (wherein X is $C_{1-4}$ alkyl).

Specific examples of pharmaceutically acceptable derivatives of the compound of formula (I) that may be used in accordance with the present invention include the monosodium salt and the following 5' esters: monophosphate; disodium monophosphate; diphosphate; triphosphate; acetate; 3-methyl-butyrate; octanoate; palmitate; 3-chloro benzoate; benzoate; 4-methyl benzoate; hydrogen succinate; pivalate; and mesylate.

The compounds according to the invention, also referred to herein as the active ingredient, may be administered for therapy by any suitable route including oral, rectal, nasal, topical (including buccal and sublingual), vaginal and parenteral (including subcutaneous, intramuscular, intravenous and intradermal). It will be appreciated that the preferred route will vary with the condition and age of the recipient, the nature of the infection and the chosen active ingredient.

In general a suitable dose will be in the range of 3.0 to 120 mg per kilogram body weight of the recipient per day, preferably in the range of 6 to 90 mg per kilogram body weight per day and most preferably in the range 15 to 60 mg per kilogram body weight per day. The desired dose is preferably presented as two, three, four, five, six or or more sub-doses administered at appropriate intervals throughout the day. These sub-doses may be administered in unit dosage forms, for example, containing 10 to 1500 mg, preferably 20 to 1000 mg, and most preferably 50 to 700 mg of active ingredient per unit dosage form.

Experiments with 3'-azido-3'-deoxythymidine suggest that a dose should be administered to achieve peak plasma concentrations of the active compound of from about 1 to about 75 $\mu$M, preferably about 2 to 50 $\mu$M, most preferably about 3 to about 30 $\mu$M. This may be achieved, for example, by the intravenous injection of a 0.1 to 5% solution of the active ingredient, optionally in saline, or orally administered as a bolus containing about 1 to about 100 mg/kg of the active ingredient. Desirable blood levels may be maintained by a continuous infusion to provide about 0.01 to about 5.0 mg/kg/hour or by intermittent infusions containing about 0.4 to about 15 mg/kg of the active ingredient.

While it is possible for the active ingredient to be administered alone it is preferable to present it as a pharmaceutical formulation. The formulations of the present invention comprise at least one active ingredient, as above defined, together with one or more acceptable carriers thereof and optionally other therapeutic agents. Each carrier must be "acceptable" in the sense of being compatible with the other ingredients of the formulation and not injurious to the patient. Formulations include those suitable for oral, rectal, nasal, topical (including buccal and sublingual), vaginal or parenteral (including subcutaneous, intramuscular, intravenous and intradermal) administration. The formulations may conveniently be presented in unit dosage form and may be prepared by any methods well known in the art of pharmacy. Such methods include the step of bringing into association the active ingredient with the carrier which constitutes one or more accessory ingredients. In general, the formulations are prepared by uniformly and intimately bringing into association the active ingredient with liquid carriers or finely divided solid carriers or both, and then if necessary shaping the product.

Formulations of the present invention suitable for oral administration may be presented as discrete units such as capsules, cachets or tablets each containing a predetermined amount of the active ingredient; as a powder or granules; as a solution or a suspension in an aqueous or non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder (e.g. povidone, gelatin, hydroxypropylmethyl cellulose), lubricant, inert diluent, preservative, disintegrant (e.g. sodium starch glycollate, cross-linked povidone, cross-linked sodium carboxymethyl cellulose) surface-active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein using, for example, hydroxypropylmethyl cellulose in varying proportions to provide the desired release profile.

Formulations suitable for topical administration in the mouth include lozenges comprising the active ingredient in a flavoured basis, usually sucrose and acacia or tragacanth; pastilles comprising the active ingredient in an inert basis such as gelatin and glycerin, or sucrose and acacia; and mouthwashes comprising the active ingredient in a suitable liquid carrier.

Formulations for rectal administration may be presented as a suppository with a suitable base comprising for example cocoa butter or a salicylate.

Formulations suitable for vaginal administration may be presented as pessaries, tampons, creams, gels, pastes, foams or spray formulations containing in addition to the active ingredient such carriers as are known in the art to be appropriate.

Formulations suitable for parenteral administration include aqueous and non-aqueous isotonic sterile injection solutions which may contain anti-oxidants, buffers, bacteriostats and solutes which render the formulation isotonic with

the blood of the intended recipient; and aqueous and non-aqueous sterile suspensions which may include suspending agents and thickening agents. The formulations may be presented in unit-dose or multi-dose sealed containers, for example, ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage formulations are those containing a daily dose or unit, daily sub-dose, as herein above recited, or an appropriate fraction thereof, of an active ingredient.

The compounds according to the invention may also be presented for use in the form of veterinary formulations, which may be prepared, for example, by methods that are conventional in the art. Examples of such veterinary formulations include those adapted for:-

(a) oral administration, for example drenches (e.g. aqueous or non-aqueous solutions or suspensions); tablets or boluses; powders, granules or pellets for admixture with feed stuffs; pastes for application to the tongue;

(b) parenteral administration for example by sub-cutaneous, intramuscular or intravenous injection e.g. as a sterile solution or suspension; or (when appropriate) by intramammary injection where a suspension or solution is introduced into the udder via the teat;

(c) topical application, e.g. as a cream, ointment or spray applied to the skin; or

(d) intravaginally, e.g. as a pessary, cream or foam.

The administered ingredients may also be used in therapy in conjunction with other medicaments such as 9-[[2-hydroxy-1-(hydroxy-methyl)ethoxy]methyl]guanine, 9-(2-hydroxyethoxymethyl)guanine (acyclovir), 2-amino-9-(2-hydroxyethoxymethyl)purine, interferon, e.g., a interferon, interleukin II, and phosphonoformate, or in conjunction with other immune modulating therapy including bone marrow or lymphoctye transplants or medications such as levamisol or thymosin which would increase lymphocyte numbers and/or function as is appropriate.

It should be understood that in addition to the ingredients particularly mentioned above the formulations of this invention may include other agents conventional in the art having regard to the type of formulation in question, for example, those suitable for oral administration may include such further agents as sweeteners, thickeners and flavouring agents.

The compounds of formula (I) and their pharmaceutically acceptable derivatives may be prepared in conventional manner, for example as described in the following references, or by methods analogous thereto:J.R. Horwitz et al., J. Org. Chem. 29, (July 1964) 2076-78; M. Imazawa et al., J. Org. Chem, 43 (15) (1978) 3044-3048; K.A. Watanabe et al., J. Org. Chem., 45, 3274 (1980); and R.P. Glinski et al., J. Chem. Soc. Chem. Commun., 915 (1970) and A, Matsuda et al, J. Org. Chem. 45, 3274 (1980), as well as the processes described in the Examples.

A compound of formula(I) may be converted into a pharmaceutically acceptable phosphate or other ester by reaction with respectively a phosphorylating agent, e.g. $POCl_3$ or an appropriate esterifying agent, e.g. an acid halide or anhydride. The compound of formula (I), including esters thereof, may be converted into pharmaceutically acceptable salts thereof in conventional manner, e.g. by treatment with an appropriate base. An ester or salt of a compound of formula (I) may be converted into the parent compound, e.g. by hydrolysis.

The following Examples are intended for illustration only. The term'active ingredient' as as used in the Examples means a compound of formula (I) or a pharmaceutically acceptable derivative thereof.

Example 1: Tablet Formulations

The following formulations A and B were prepared by wet granulation of the ingredients with a solution of povidone, followed by addition of magnesium stearate and compression.

| Formulation A | | |
|---|---|---|
| | mg/tablet | mg/tablet |
| (a) Active ingredient | 250 | 250 |
| (b) Lactose B.P. | 210 | 26 |
| (c) Povidone B.P. | 15 | 9 |
| (d) Sodium Starch Glycollate | 20 | 12 |

Continuation of the Table on the next page

(continued)

| Formulation A | | |
|---|---|---|
| | mg/tablet | mg/tablet |
| (e) Magnesium Stearate | 5 | 3 |
| | 500 | 300 |

| Formulation B | | |
|---|---|---|
| | mg/tablet | mg/tablet |
| (a) Active ingredient | 250 | 250 |
| (b) Lactose | 150 | - |
| (c) Avicel PH 101 | 60 | 26 |
| (d) Povidone B.P. | 15 | 9 |
| (e) Sodium Starch Glycollate | 20 | 12 |
| (f) Magnesium Stearate | 5 | 3 |
| | 500 | 300 |

| Formulation C- | |
|---|---|
| | mg/tablet |
| Active ingredient | 100 |
| Lactose | 200 |
| Starch | 50 |
| Povidone | 5 |
| Magnesium stearate | 4 |
| | 359 |

The following formulations, D and E, were prepared by direct compression of the admixed ingredients. The lactose used in formulation E was of the direct compression type (Dairy Crest - "Zeparox").

| Formulation D | |
|---|---|
| | mg/capsule |
| Active ingredient | 250 |
| Pregelatinised Starch NF15 | 150 |
| | 400 |

| Formulation E | |
|---|---|
| | mg/capsule |
| Active Ingredient | 250 |
| Lactose | 150 |
| Avicel | 100 |
| | 500 |

Formulation F (Controlled Release Formulation)

The formulation was prepared by wet granulation of the ingredients (below) with a solution of povidone followed by

the addition of magnesium stearate and compression.

| | mg/tablet |
|---|---|
| (a) Active Ingredient | 500 |
| (b) Hydroxypropylmethylcellulose (Methocel K4M Premium) | 112 |
| (c) Lactose B.P. | 53 |
| (d) Povidone B.P.C. | 28 |
| (e) Magnesium Stearate | 7 |
| | 700 |

Drug release took place over a period of about 6-8 hours and was complete after 12 hours.

Example 2: Capsule Formulations

Formulation A

A capsule formulation was prepared by admixing the ingredients of Formulation D in Example 1 above and filling into a two-part hard gelatin capsule. Formulation B (infra) was prepared in a similar manner.

Formulation B

| | mg/capsule |
|---|---|
| (a) Active ingredient | 250 |
| (b) Lactose B.P. | 143 |
| (c) Sodium Starch Glycollate | 25 |
| (d) Magnesium Stearate | 2 |
| | 420 |

Formulation C

| | mg/capsule |
|---|---|
| (a) Active ingredient | 250 |
| (b) Macrogol 4000 BP | 350 |
| | 600 |

Capsules were prepared by melting the Macrogol 4000 BP, dispersing the active ingredient in the melt and filling the melt into a two-part hard gelatin capsule.

Formulation D

| | mg/capsule |
|---|---|
| Active ingredient | 250 |
| Lecithin | 100 |
| Arachis Oil | 100 |
| | 450 |

Capsules were prepared by dispersing the active ingredient in the lecithin and arachis oil and filling the dispersion into

soft, elastic gelatin capsules.

Formulation E (Controlled Release Capsule)

The following controlled release capsule formulation was prepared by extruding¸ ingredients a, b and d using an extruder, followed by spheronisation of the extrudate and drying. the dried pellets were then coated with release- controlling membrane (d) and filled into a two-piece, hard gelatin capsule.

| | mg/capsule |
|---|---|
| (a) Active Ingredient | 250 |
| (b) Microcrystalline Cellulose | 125 |
| (c) Lactose BP | 125 |
| (d) Ethyl Cellulose | 13 |
| | 513 |

Example 3: Injectable Formulation

| Formulation A. | |
|---|---|
| Active ingredient | 0.200g |
| Hydrochloric acid solution, 0.1M q.s. to pH | 4.0 to 7.0 |
| Sodium hydroxide solution, 0.1M q.s. to pH | 4.0 to 7.0 |
| Sterile water q.s. to | 10ml |

The active ingredient was dissolved in most of the water (35°-40°C) and the pH adjusted to between 4.0 and 7.0 with the hydrochloric acid or the sodium hydroxide as appropriate. The batch was then made up to volume with the water and filtered through a sterile micropore filter into a sterile 10ml amber glass vial (type 1) and sealed with sterile closures and overseals.

| Formulation B- | |
|---|---|
| Active ingredient | 0.125 g |
| Sterile, pyrogen-free, pH 7 phosphate buffer, q.s. to | 25 ml |

Example 4: Intramuscular injection

| | |
|---|---|
| Active Ingredient | 0.20 g |
| Benzyl Alcohol | 0.10 g |
| Glycofurol 75 | 1.45 g |
| Water for Injection q.s. to | 3.00 ml |

The active ingredient was dissolved in the glycofurol. The benzyl alcohol was then added and dissolved, and water added to 3 ml. The mixture was then filtered through a sterile micropore filter and sealed in sterile 3 ml amber glass vials (type 1).

Example 5: Syrup

| | |
|---|---|
| Active ingredient | 0.2500 g |

Continuation of the Table on the next page

(continued)

| | |
|---|---|
| Sorbitol Solution | 1.5000 g |
| Glycerol | 2.0000 g |
| Sodium Benzoate | 0.0050 g |
| Flavour, Peach 17.42.3169 | 0.0125 ml |
| Purified Water q.s. to | 5.0000 ml |

The active ingredient was dissolved in a mixture of the glycerol and most of the purified water. An aqueous solution of the sodium benzoate was then added to the solution, followed by addition of the sorbitol solution and finally the flavour. The volume was made up with purified water and mixed well.

Example 6: Suppository

| | mg/suppository |
|---|---|
| Active Ingredient (63μm)* | 250 |
| Hard Fat, BP (Witepsol H15 - Dynamit NoBel) | 1770 |
| | 2020 |

*The active ingredient was used as a powder wherein at least 90% of the particles were of 63μm diameter or less.

One-fifth of the Witepsol H15 was melted in a steam-jacketed pan at 45°C maximum. The active ingredient was sifted through a 200μm sieve and added to the molten base with mixing, using a silverson fitted with a cutting head, until a smooth dispersion was achieved. Maintaining the mixture at 45°C, the remaining Witepsol H15 was added to the suspension and stired to ensure a homogenous mix. The entire suspension was passed through a 250μm stainless steel screen and, with continuous stirring, was allowed to cool to 40°C. At a temperature of 38°C to 40°C, 2.02g of the mixture was filled into suitable, 2 ml plastic moulds. The suppositories were allowed to cool to room temperature.

Example 7: Pessaries

| | mg/pessary |
|---|---|
| Active ingredient (63μm) | 250 |
| Anhydrate Dextrose | 380 |
| Potato Starch | 363 |
| Magnesium Stearate | 7 |
| | 1000 |

The above ingredients were mixed directly and pessaries prepared by direct compression of the resulting mixture.

Example 8: 3'-Azido-3'-deoxy-5'-0-octanoylthymidine

To a solution of 3'-azido-3'-deoxy thymidine in pyridine (0°C), octanoyl chloride (1.2 equivalents) was added. The reaction was allowed to warm to room temperature.

When tlc ($CHCl_3$:MeOH;20:1, on silica gel) indicated complete reaction, the solution was poured onto ice water. The aqueous phase was decanted. The resulting oil was chromatographed on silica gel eluted with $CHCl_3$:MeOH. The title compound was obtained as an oil by evaporation of the solvent from the appropriate fractions.

CHN:cal. C-54.95 H-6.92 N-17.80

fnd. C-54.82 H-6.96 N-17.66

δ7.46(d,1H,$J_{5,6}$=1Hz,6H), δ6.13(t,1H,1'H), δ4.5-4.2(m,3H,3'H and 5'$CH_2$) δ4.0-3.8(m,1H,4'H), δ2.3-2.1(m,4H,2'H and ($CH_2$)l of octanoyl)), δ1.81(d,3H,$J_{5,6}$=1.0Hz, 5$CH_3$), δ1.5-0.6(m,13H,5' octanoyl $(CH_2)_5CH_3$)

Example 9: 5'-Acetyl-3'-azido-3'-deoxythymidine

To a solution of 3'-azido-3'-deoxythymidine (20g) in pyridine (50ml) at ambient temperature, acetyl chloride (2.1 equivalents) was added. The reaction was stirred for two hours and kept at 0 to 5°C for 20 hours. It was poured onto ice water with stirring. The aqueous phase was decanted. The oily product was dissolved in water and extracted with water (5 times), 0.5 N hydrochloric acid, water (2x), and dried over magnesium sulphate. The solution was filtered and evaporated in vacuo. The residual oil was dissolved in chloroform, applied to a silica gel column, and flash chromatographed using 2% methanol in chloroform. Fractions with product were evaporated and the oil was chromatographed again using ethyl acetate: hexane (6:4 v/v).
m.p. 96-98° C
cal. C-46.60 H-4.89 N-22.65
fnd. C-46.67 H.4.94 N-22.59

Example 10

The following compounds were prepared according to the procedure of Example 8 or 9 as appropriate from the appropriate acid halide or anhydride.

| | |
|---|---|
| 3'-Azido-5'-O-benzoyl-3'-deoxythymidine | cal. C-53.68 H-4.77 N-18.41<br>fnd. C-53.81 H-4.72 N-18.46<br>m.p. 54-59 °C |
| 3'-Azido-3'-deoxy-5'-O-pivaloylthymidine | cal. C-51.27 H-6.03 N-19.93<br>fnd. C-51.07 H-6.05 N-19.83<br>m.p. 99-100 °C |
| 3'-Azido-3'-deoxy-5'-O-(3-methylbutyryl)thymidine | cal. C-50.24 H-6.13 N-19.53<br>fnd. C-50.27 H-6.11 N-19.49 |

δ7.46(d,1H,$J_{5,6}$=1.2Hz,6H), δ6.13(t,1H,1'H), δ4.55-4.15(m,3H,3'H and 5'CH2), δ3.8-4.15(m,1H,4'H), δ2.4-1.78(m,3H,2'H and 5'methine), δ1.80(d,3H,$J_{5,6}$=1.2Hz,5$CH_3$), δ0.9(d,6H,J=6.4Hz, methyls on 5'butyryl)

| | |
|---|---|
| 3'-Azido-3'-deoxy-5'-O-palmitoylthymidine | cal. C-61.67 H-8.57 N-13.85<br>fnd. C-61.85 H-8.59 N-13.75 |

δ7.45(d,1H,$J_{5,6}$=1.0Hz,6H), δ6.12(t,1H,1'H), δ4.5-4.05(m,3H,3'H and 5'$CH_2$), δ4.0-3.8(m,1H,4'H) δ2.35-2.11(m,4H,2'H and ($CH_2$)1 of palmitoyl), δ1.8 d, 3H, $J_{5,6}$=1.0Hz,5$CH_3$) δ1.35-0.6(m,29H,5'palmitoyl$(CH_2)_{13}CH_3$)

3'-Azido-3'-deoxy-5'-O-toluylthymidine

Cal. C-56.10; H-4.97; N-18.17
Fnd. C-55.88; H-5.00; N-18.09
m.p. 73°C

NMR taken in DMSO-$d_6$
NMR: δ7.95-7.29 (m,5H; bH,cH,6H), δ6.16 (t,1H,1'H), δ4.6-4.4 (m,3H,3'H,5'H), δ4.2-4.0 (m,1H,4'H), δ2.39 (s,3H,d$CH_3$), δ1.63 (s,3H,5$CH_3$)

3'-Azido-3'-deoxythymidine 5'-O-(hydrogen succinate)

Cal. C-44.98; H-4.83; N-17.96
Fnd. C-44.90; H-4.77; N-17.85

NMR taken in DMSO-$d_6$
NMR: δ7.46 (s,1H,6H), δ6.13 (m,1H,1'H), δ4.48-4.40 (m,1H,3'H), δ4.34-4.20 (m,2H,5'H), δ3.99-3.94 (m,1H,4'H), δ1.78 (s,3H,5$CH_3$)

3'-Azido-3'-deoxy-5'-mesylthymidine

Cal. C-38.25; H-4.37; N-20.28; S-9.28
Fnd. C-38.15; H-4.38; N-20.19:S-9.30
m.p. 253°C (dec.)

NMR taken in DMSO-$d_6$
NMR: δ7.49 (d,1H,$J_{6,5}$ = 1.0 Hz, 6H), δ6.15 (t,1H,$J_{1',2'}$ = 6.6 Hz, 1'H), δ4.54-4.41 (m,3H;3'H,5'H), δ4.14-4.02 (m,1H,4'H), δ3.24 (s,3H,5'-mesyl $CH_3$), δ1.79 (d, 3H,$J_{5,6}$ = 1.0 Hz, 5$CH_3$)

3'-Azido-5'-O-(3-chlorobenzoyl)-3'-deoxythymidine

Cal. C-50.31; H-3.97; N-17.26; Cl-8.74
Fnd. C-50.16; H-4.03; N-17.13; Cl-8.66

NMR taken in DMSO-$d_6$
NMR: δ11.37 (s,1H,3-NH), δ7.98-7.43 (m,5H;5'-phenyl,6H), δ6.17 (dd,1H; $J_{1',2a'}$ = 6.1 Hz, $J_{1',2b'}$ = 7.2 Hz, 1'H), δ4.68-4.48 (m,3H;3'H,5'H), δ4.14-4.11 (m,1H,4'H), δ2.48-2.41 (m,2H,2'H), δ1.64 (d,3H,$J_{5,6}$ = 1.2 Hz, 5$CH_3$)

Example 11: 2,5'-0-Anhydro-3'-azido-3'-deoxythymidine

3'-Azido-3'-deoxythymidine (3.0g, 11.2 mMol) was mesylated by the addition of methanesulphonyl chloride (2.7 mL) to a solution of the starting material in dry pyridine (2 mL). The reaction was allowed to proceed at 5°C for one hour, then poured onto ice water. The precipitate was collected by filtration. The product (3'-azido-5'-mesylthymidine) was reacted with potassium carbonate (0.78 g, 5.6 mMol) in DMF (75 mL). The reactants were heated in an 80°C oil bath for six hours, then poured into ice water. The product was extracted from the water with ethyl acetate. The solvent was removed in vacuo and the resultant oil was flash chromatographed on silica gel by elution with $CHCl_3$:MeOH (9:1 v/v). The title compound was obtained as a solid after evaporation of the solvent from the appropriate fractions.
m.p. = 184 - 186°C

Example 12: 3'-Azido-3'-deoxythymidine

a) 2,3'-Anhydrothymidine

Thymidine (85.4 g: 0.353 mol) was dissolved in 500 ml dry DMF and added to N-(2-chloro-1,1,2-trifluoroethyl)diethylamine (100.3 g; 0.529 mol) (prepared according to the method of D.E. Ayer, J. Med. Chem. 6, 608 (1963)). This solution was heated at 70°C for 30 minutes then poured into 950 ml ethanol (EtOH) with vigorous stirring. The product precipitated from this solution and was filtered. The EtOH supernatant was refrigerated then filtered to yield the title compound. mp. = 228 -230 ° C.

b) 3'-Azido-3'-deoxythymidine

2,3'-0-Anhydrothymidine (25 g: 0.1115 mol) and $NaN_3$ (29 g, 0.446 mol) was suspended in a mixture of 250 ml DMF and 38 ml water. The reaction mixture was refluxed for 5 hours at which time it was poured into 1 liter of water. The aqueous solution was extracted with EtOAc (3 x 700 ml). The EtOAc extracts were dried over $Na_2SO_4$, filtered and the EtOAc was removed in vacuo to yield a viscous oil. This oil was stirred with 200 ml water providing the title compound as a solid which was collected by filtration. mp = 116-118°C

Example 13: Monosodium Salt of 3'-Azido-3'-deoxythymidine

Approximately 1g of 3'-azido-2',3'-dideoxythymidine was dissolved in 50mL of distilled water. The pH was adjusted to 12 with 1N NaOH. Approximately half of the solution was freeze-dried. The title compound was obtained as a lyophilised powder.
Analysis calculated for $C_{10}H_{12}N_5NaO_4$ 6/10 $H_2O$
cal: C-40.03; H-4.43; N-23.34; Na-7.66
fnd: C-39.88; H-4.34; N-23.23; Na-7.90

In Vitro Antibacterial Activity

Table 1 demonstrates the in vitro antibacterial activity of the compound 3'-azido-3'-deoxythymidine in terms of its Minimum Inhibitory Concentration (MIC) against various bacterial species.
The standard used was trimethoprim (TMP).
The medium used was Wellcotest Sensitivity Test Agar plus 7% lysed horse blood.

Table 1

| | MIC(µg/ml) | | |
|---|---|---|---|
| Organism | Compound | | Standard |
| Salmonella typhimurium (LT-2) | S8587 | 0.01 | 0.1-0.5 |
| Salmonella typhosa | CN512 | 0.005 | 0.03-0.1 |
| Shig.flexneri | CN6007 | 0.05 | 0.03-0.1 |
| Kleb.pneumoniae | CN3632 | 0.5 | 0.3-1.0 |
| Entero.aerogenes | 2200/86 | 0.005 | 0.3-0.5 |
| Citrofreundii | 2200/77 | 0.005 | 0.3-0.5 |
| Pr.vulgaris | CN329 | 0.5 | 0.5-1.0 |
| E.coli (R483) | S4362 | 0.005 | >50-100 |
| E.coli(R67) | S6137 | 0.005 | >50-100 |

In Vivo Activity Against S.dublin Infection in Calves

Three calves were infected with Salmonella dublin on Day 0, and then on Days 2, 3 and 4 the calves were given subcutaneous injections of a solution of 3'-azido-3'-deoxythymidine (in a mixture of dimethylformamide and water) at 30, 15 or 7.5 mg/kg. All the calves responded to treatment, and although the two receiving the lower doses showed a slight relapse on days 6 and 7, they all recovered completely. No drugresistant S.dublin were recovered from the

post-treatment rectal swabs.

Toxicity Assay

3'-Azido-3'-deoxythymidine was administered to both mice and rats. The $LD_{50}$ value was in excess of 750 mg/kg in both species.

Anti-Feline Leukaemia Activity of 3'-Azido-3'-deoxythymidine

(i) In Vitro Activity

Susceptible feline embryo lung fibroblasts (FLF-3) were seeded onto multiwell slides ($10^5$ cells/ml, 0.05ml/well) with Dulbecco-modified Eagle's essential medium (DME) and incubated at 37°C overnight. Each of the 32 wells was then infected with 40-60 focus-forming units (ffu) of feline leukaemia virus (FeLV) for one hour after which the medium was replaced with fresh DME with varying concentrations of 3'-azido-3'-deoxythymidine per 4 wells. Concentrations were 0, 0.1, 1.0, 10, 50, 100, 200 and 30µM. After 3 days incubation at 37°C, the cultures were assayed by the indirect fluorescent antigen test for the production of FeLV. Complete absence of FeLV was seen at concentrations of 50µM and above, demonstrating total efficacy at these levels of drug. At 10µM, 90% inhibition was seen, at 1µm, 50-75% inhibition, and no activity was noted at 0.1µm 3'-azido-3'-deoxythymidine.

(ii) In Vivo Activity

Five, otherwise healthy, cats, persistently infected with FeLV for at least 6 months, were treated with intravenous 3'-azido-3'-deoxythymidine. Two cats (a) received 15mg/kg/day, three (b) received 30mg/kg/day and another two (c) received 45mg/kg/day. All doses were administered as three equal doses daily. Of cats (a), only one responded to treatment at all, showing a 10% decrease in $FeLV^+$ white blood cells (wbc's). Cats (b) (after 6 weeks) all responded to treatment showing a 10% and 90% drop in $Fel_V^+$ wbc's. The third showed a 5% drop in $FeLV^+$ wbc's and a hundred-fold drop in plasma levels of free FeLV. Cats (c) showed, in one case, no response and, in the other, a 10% decrease in $FeLV^+$ wbc's and a marked reduction in lymphadenopathy (from 2 cm to 0.5 cm). All measurements were taken after 3 weeks therapy, unless otherwise stated.

**Claims**

**1.** A compound of formula (I):

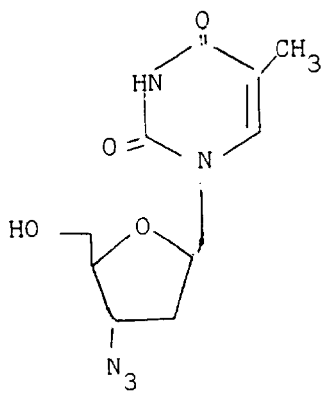

(I)

or a pharmaceutically acceptable derivative thereof, for use in therapy, other than for use in the treatment or prophylaxis of human retrovirus infections.

**2.** A compound of formula (I) as claimed in claim 1, or a pharmaceutically acceptable derivative thereof, for use in the treatment or prophylaxis of non-human animal retroviral infections.

**3.** A compound of formula (I) as claimed in claim 1, or a pharmaceutically acceptable derivative thereof, for use in the treatment or prophylaxis of a feline leukaemia virus or equine infectious anaemia virus infection.

**4.** A compound of formula (I) as claimed in claim 1, or a pharmaceutically acceptable derivative thereof, for use in the

treatment or prophylaxis of a gram-negative bacterial infection.

5. A compound of formula (I) as claimed in claim 1, or a pharmaceutically acceptable derivative thereof, for use in the treatment or prophylaxis of calf neonatal enteritis, pig post-weaning enteritis or chicken colisepticaemia.

6. A pharmaceutically acceptable derivative in the form of a salt, ester or salt of said ester of a compound of formula (I) for use as claimed in any of the preceding claims.

7. Use of a compound of formula (I) (as defined in claim 1), or a pharmaceutically acceptable derivative thereof, in the manufacture of a medicament for the treatment or prophylaxis of non-human retrovirus infections.

**Patentansprüche**

1. Verbindung der Formel (1):

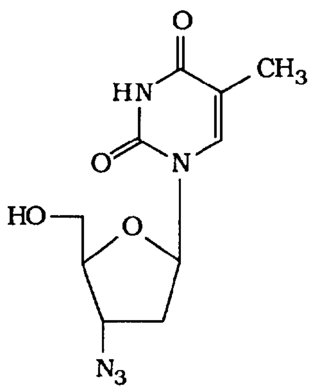

(I)

oder ein pharmazeutisch annehmbares Derivat davon zur Verwendung in der Therapie mit Ausnahme der Behandlung oder Prophylaxe von Retrovirusinfektionen des Menschen.

2. Verbindung der Formel (I) nach Anspruch 1 oder ein pharmazeutisch annehmbares Derivat davon zur Verwendung bei der Behandlung oder Prophylaxe von nicht-menschlichen Retrovirusinfektionen von Tieren.

3. Verbindung der Formel (I) nach Anspruch 1 oder ein pharmazeutisch annehmbares Derivat davon zur Verwendung bei der Behandlung oder Prophylaxe einer Leukämievirusinfektion bei Katzen oder bei einer infektiösen Anämievirusinfektion bei Pferden.

4. Verbindung der Formel (I) nach Anspruch 1 oder ein pharmazeutisch annehmbares Derivat davon zur Verwendung bei der Behandlung oder Prophylaxe einer Infektion durch gram-negative Bakterien.

5. Verbindung der Formel (I) nach Anspruch 1 oder ein pharmazeutisch annehmbares Derivat davon zur Verwendung bei der Behandlung oder Prophylaxe von Enteritis bei frischgeborenen Kälbern, Enteritis bei Schweinen nach dem Säugestadium oder von Coliblutvergiftung bei Hühnern.

6. Pharmazeutisch annehmbares Derivat in Form eines Salzes, Esters oder Salzes des Esters einer Verbindung der Formel (I) zurVerwendung nach einem der vorhergehenden Ansprüche.

7. Verwendung einer Verbindung der Formel (I) (wie sie in Anspruch 1 definiert ist) oder eines pharmazeutisch annehmbaren Derivats davon bei der Herstellung eines Arzneimittels zur Behandlung oder Prophylaxe von Retrovirusinfektionen bei vom Menschen verschiedenen Organismen.

**Revendications**

1. Composé de formule (I):

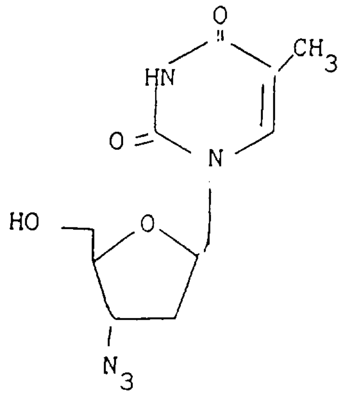

(I)

ou dérivé pharmaceutiquement acceptable de celui-ci, pour utilisation en thérapie, autre que pour utilisation dans le traitement ou la prophylaxie d'infections rétrovirales humaines.

2. Composé de formule (I) selon la revendication 1, ou dérivé pharmaceutiquement acceptable de celui-ci, pour utilisation dans le traitement ou la prophylaxie d'infections rétrovirales animales non humaines.

3. Composé de formule (I) selon la revendication 1, ou dérivé pharmaceutiquement acceptable de celui-ci, pour utilisation dans le traitement ou la prophylaxie d'une infection par le virus de la leucémie féline ou le virus de l'anémie infectieuse équine.

4. Composé de formule (I) selon la revendication 1, ou dérivé pharmaceutiquement acceptable de celui-ci, pour utilisation dans le traitement ou la prophylaxie d'une infection par bactéries Gram négatives.

5. Composé de formule (I) selon la revendication 1, ou dérivé pharmaceutiquement acceptable de celui-ci, pour utilisation dans le traitement ou la prophylaxie de l'entérite néonatale du veau, de l'entérite après sevrage du porc ou de la colisepticémie du poulet.

6. Dérivé pharmaceutiquement acceptable, sous la forme d'un sel, d'un ester ou d'un sel dudit ester, d'un composé de formule (I), pour utilisation selon l'une quelconque des revendications précédentes.

7. Utilisation d'un composé de formule (I) (tel que défini dans la revendication 1), ou d'un dérivé pharmaceutiquement acceptable de celui-ci, dans la fabrication d'un médicament pour le traitement ou la prophylaxie d'infections rétrovirales non humaines.